# EUROPEAN PATENT APPLICATION

(11) **EP 0 995 804 A2**
(43) Date of publication of application: **26.04.2000**
(21) Application number: 99250325.0
(22) Date of filing: 15.09.1999
(51) Int. Cl.: C12Q 1/68

(54) **Nucleic acid analysis using complete N-mer arrays**

(30) Priority: 15.09.1998 US 100393 P
(71) Applicant: Affymetrix, Inc. (a California Corporation), Santa Clara, CA 95051 (US)
(72) Inventor: Gunderson, Kevin L., Encinitas, CA 92024 (US); Chee, Mark S., Del Mar, CA 92014 (US); Lockhart, David J., Del Mar, CA 92014 (US)
(74) Representative: UEXKÜLL & STOLBERG

(57) **Abstract**

Methods for characterizing mutations in a polynucleotide are provided. Such methods comprise of separately ligating fragments of a reference polynucleotide and a target polynucleotide to identical or substantially identical arrays of polynucleotide probes having single-stranded overhangs constituting complete or substantially complete n-mer sets and comparing their hybridization patterns. The methods can also be used to detect the presence of polymorphisms. Methods are also provided to determine whether two or more polynucleotides of unknown sequences are identical. Further, methods are provided to enumerate and distinguish fragmcnts of polynucleotides based on their terminal sequences. These methods are useful in medicine, pharmacogenomics, biochemistry, and forensic sciences.

## Description

### RELATED APPLICATION

This application claims priority from the U.S. Provisional Application No. 60/100,393, filed September 15, 1998, the contents of which are hereby incorporated by reference.

### FIELD OF THE INVENTION

The present invention relates to analysis of polynucleotide sequences. The invention therefore relates to diverse fields such as molecular biology, biochemistry, chemistry, medicine, and medical diagnostics.

### BACKGROUND ART

The goal of genome sequencing projects is to obtain the complete sequence of a reference genome. The next, and perhaps more difficult step is to analyze sequence variation and relate this information to important phenotypes. High-density arrays of single-stranded polynucleotide probes (Fodor et al., *Nature* 364: 555-6, 1993; Pease et al., *Proc. Natl. Acad. Sci. U.S.A.* 91: 5022-6, 1994) offer a highly parallel and scalable approach to large-scale polynucleotide sequence analysis (Lipshutz, *J. Biomol. Struct. Dyn.* 11: 637-53, 1993). The utility of DNA arrays for genetic analysis has been previously demonstrated in numerous applications including mutation detection, genotyping, physical mapping and gene-expression monitoring (Chee et al., *Science* 274: 610-14, 1996; Lockhart et al., *Nat. Biotech.* 14: 1675-80, 1996; Cronin et al., *Hum. Mutat.* 7: 244-55, 1996; Kozal et al., *Nat. Med.* 2: 753-59, 1996; Wodicka et al., *Nat. Biotech.* 15: 1359-72, 1997).

In some assay formats, arrays of single-stranded polynucleotide probes immobilized on solid supports have been used to detect specific polynucleotide sequences in a target polynucleotide. See, *e.g.,* PCT patent publication Nos. WO 89/10977 and 89/11548. The development of VLSIPS^{TM} technology has provided methods for making very large arrays of single-stranded polynucleotide probes in very small areas. See U.S. Pat. No. 5,143,154 and PCT patent publication Nos. WO 90/15070 and 92/10092, each of which is incorporated herein. U.S. Pat. No. 5,837,832 describes methods for microfabricating arrays of large numbers of single-stranded polynucleotide probes, called "DNA chips," in which the probes have specific sequences at predetermined locations in the array to facilitate identification of a specific target polynucleotide, or to detect whether one or more specific sequences of a target polynucleotide in a sample varies from a previously characterized sequence.

Most of the arrays of single-stranded polynucleotide probes used successfully to date have employed customized sets of probes specifically prepared based upon predefined reference sequences. A more general, efficient and faster approach to array-based sequence determination and genetic analysis (*i.e.,* mutation and polymorphism analysis) might be to use complete sets of single-stranded polynucleotide probes for a given length ("n-mer sets") so that, in principle, any target sequence can be queried, provided that existing problems with this technique can be surmounted.

Major hurdles exist in the development of complete n-mer probe sets of single-stranded polynucleotides. First, the total number of probes for a complete set of n-mers (4ⁿ) can be very Large as "n" increases. Only recently have technological advancements been made to facilitate the synthesis of large number of nucleic acid probe arrays.

Additionally, even though several theoretical proposals have been put forth describing DNA sequence reconstruction based upon hybridization to complete sets of n-mer probes (see, Bains and Smith, *J. Theor. Biol.* 135: 303-7, 1988; Lysov Iu et al., *Dokl. Akad. Nauk SSSR* 303: 1508-11, 1988; Drmanac et al., *Genomics* 4: 114-28, 1989), the methods have been unsuccessful. Sequence reconstruction has been hampered by ambiguities resulting from the presence of sequence repeats and lack of n-mer uniqueness in a complex polynucleotide. Additional problems include a shortage of computational and analytical methods to handle inevitably imperfect hybridization data.

U.S. Pat. No. 5,503,980 describes arrays of polynucleotides having a constant double-stranded region and a variable single-stranded region for sequence determination and analysis. The '980 method employed hybridization of target polynucleotides to the probe array. The hybridization was followed by ligation. However, the '980 patent methodology involves many steps, is cumbersome, and is not standardized to allow many different samples to be tested on a single array of probes.

For example, the methods of the '980 patent required using a reference polynucleotide to customize a probe array, then using that customized probe array to detect mutations in target polynucleotides. As a result, the '980 methodology produces a probe array that is useful for detecting mutations in only the target polynucleotide for which it is designed. Further, the methodology can detect only single substitution mutations (not insertions or deletions), and can neither characterize the mutation it detects nor identify the mutation's position in the nucleotide sequence.

Methods of sequence analysis (*e.g,* characterizing mutations, detection of polymorphic sequences, etc.,) that would avoid the inherent difficulties of sequencing-by-hybridization while retaining the advantages of complete n-mer arrays are desirable. In particular, a complete set of standardized arrays of polynucleotides of a given length, without the necessity of constructing specific arrays for each target, is highly desirable. Such standardized array methodology allows direct, accurate and efficient sequence analysis, including characterization of mutations and detection of polymorphic sequence variations, in a target polynucleotide from a variety of sources. The present invention meets these needs.

### DISCLOSURE OF THE INVENTION

A method of determining the presence of a mutation in a target polynucleotide is provided. The method comprises the steps of:
(a) providing at least two identical or substantially identical polynucleotide probe arrays, wherein each probe comprises a double stranded region and a single-stranded n-mer overhang region such that the overhangs in each array constitute a complete or substantially complete set of n-mers;
(b) hybridizing the target polynucleotide to said overhangs of probe polynucleoides in one array to generate a target hybridization pattern;
(c) hybridizing a reference polynucleotide to said overhangs of probe polynucleotides in a second array to generate a reference hybridization pattern; and
(d) determining the presence of a mutation in the target polynucleotide by comparing the reference and target hybridization patterns.

A method is also provided to determine whether two or more target polynucleotides are identical, comprising the steps of:
(a) providing at least two identical or substantially identical polynucleotide probe arrays, wherein each probe comprises a double stranded region and a single-stranded n-mer overhang region such that the overhangs in each array constitute a complete or substantially complete set of n-mers;
(b) hybridizing first target polynucleotide to said overhangs of probe polynucleotides in one array to generate a first hybridization pattern;
(c) hybridizing second target polynucleotide to said overhangs of probe polynucleotides in a second array to generate a second hybridization pattern; and
(d) comparing the first and second hybridization patterns.

Additionally, a method is provided to distinguish and enumerate individual polynucleotides in a mixture of polynucleotides comprising the steps of:
(a) providing at least two identical or substantially identical polynucleotide probe arrays, wherein each probe comprises a double stranded region and a single-stranded n-mer overhang region such that the overhangs in each array constitute a complete or substantially complete set of n-mers;
(b) hybridizing the polynucleotides in the mixture to said overhangs of probe polynucleotides in the array; and
(c) determining the hybridization pattern.

### BRIEF DESCRIPTION OF THE DRAWINGS

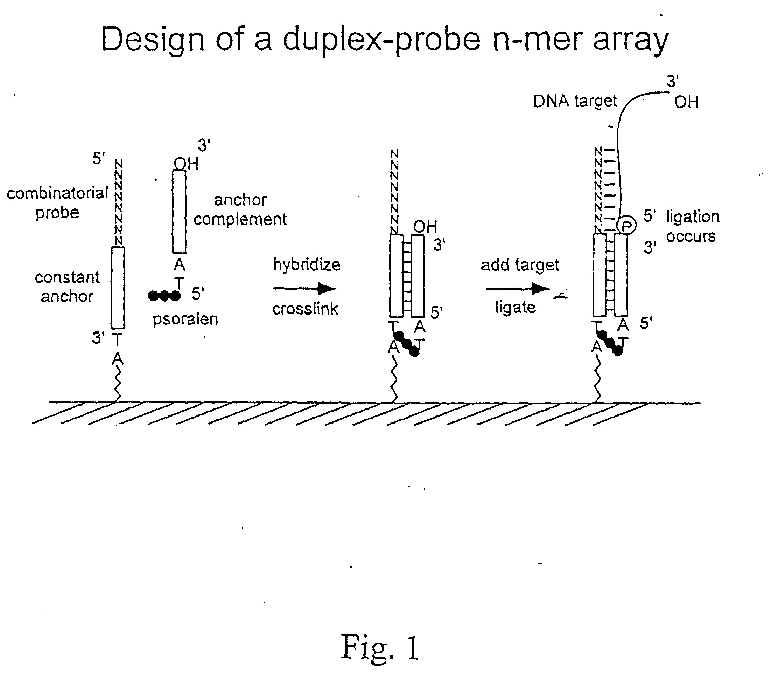

Figure 1 shows the basics of the design of an n-mer array. For illustration, a double-stranded constant anchor region with a 5' single-stranded overhang is shown. The Figure also shows an optional covalent crosslinking step (here illustrated with psoralen).

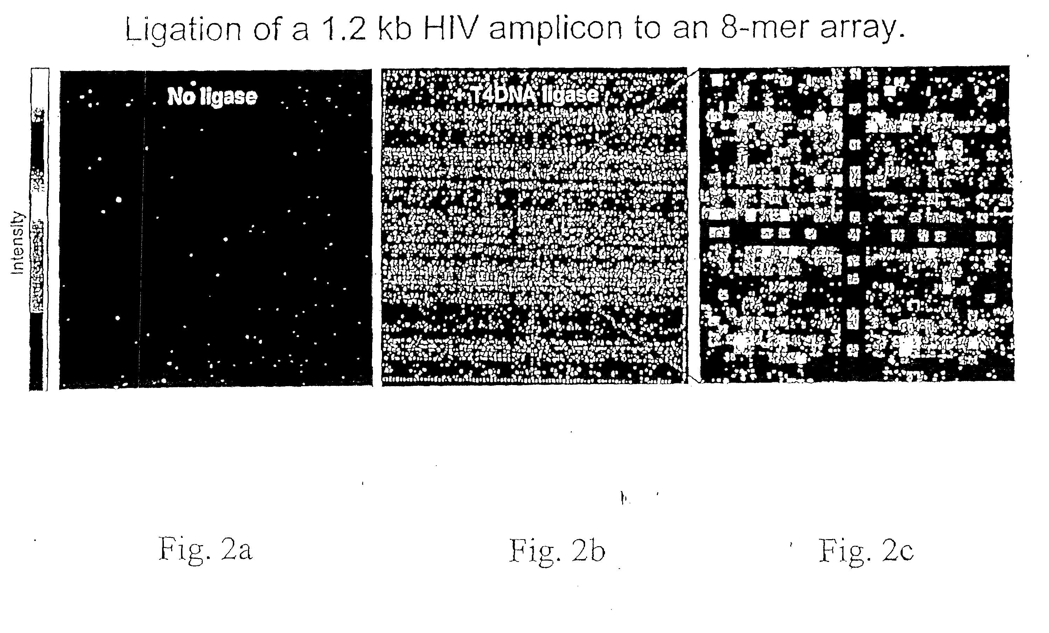

Figure 2 shows the effects of ligation of a 1.2 kb HIV amplicon to an 8-mer array. Figure 2a shows hybridization in the absence of a ligase. Figure 2b shows hybridization and ligation in the presence of a T4 DNA ligase. Figure 2c shows an expanded view of Figure 2b to illustrate the individual probe features.

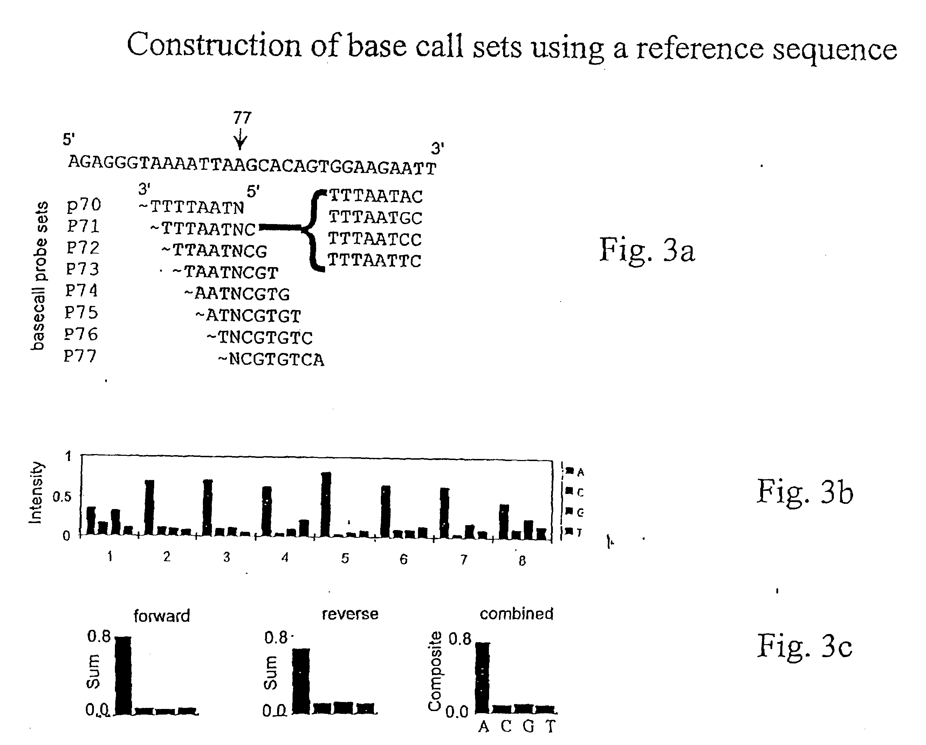

Figure 3 shows construction of base call sets using a reference sequence. Figure 3a shows an 8-mer array to which a portion of the reference sequence (forward strand) of a 540 bp DNA target is ligated. The probe sequences of the 8 base call sets interrogating position 77 within the sequence are indicated. Each base call set consists of four probes differing at single substitution position (indicated by the letter N), located so as to interrogate the specified target base.

Figure 3b shows the intensities of the probes within each base call set as extracted from the array data and plotted. The probes are enumerated such that an A-probe actually contains a complementary "T" base substitution (forward strand query) or an "A" base substitution (reverse strand query).

Figure 3c illustrates a summation of the interrogation probes (A,C,G,T) across all eight base call sets. The summation yields a summed base call set for both the forward and reverse strands. A composite base call set derived from these summed sets allows a final highly-accurate base call to be made.

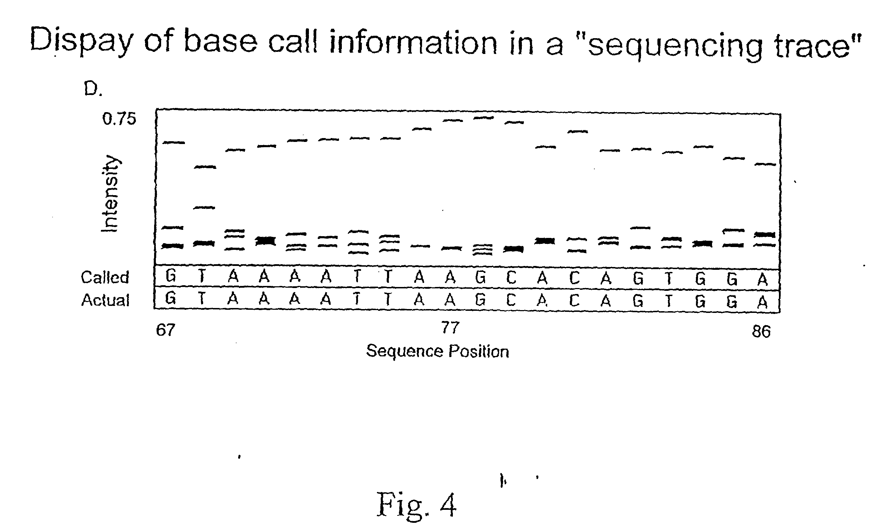

Figure 4 shows a display of base call information in a "sequencing trace."

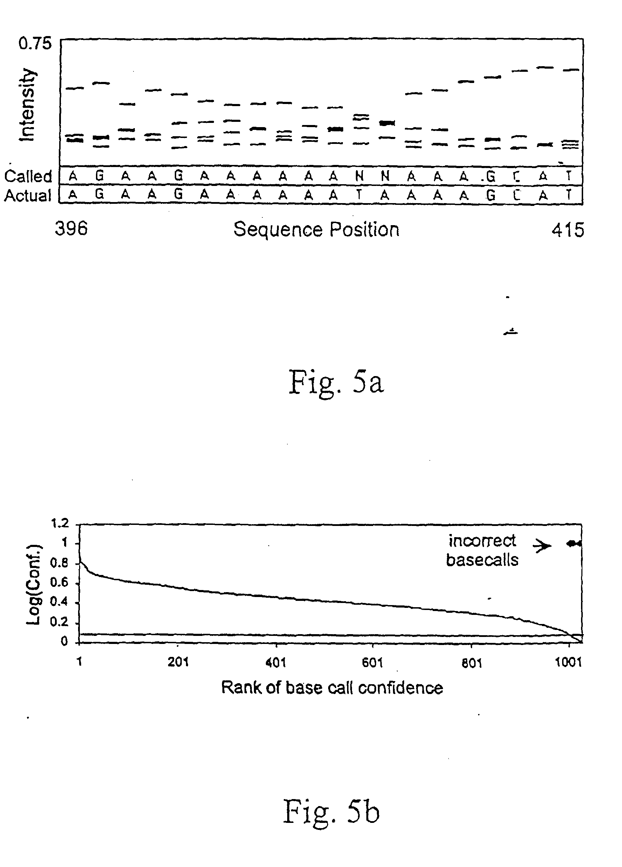

Figure 5 shows how to use base-call quality to provide a cutoff point for no-calls. Figure 5a is an example of a target region exhibiting low quality base calls (inadequate separation between intensity values), demonstrated by the sequence trace for an AT-rich region within the HIV target sequence. Figure 5b illustrates that cutoff threshold eliminated several incorrect base calls.

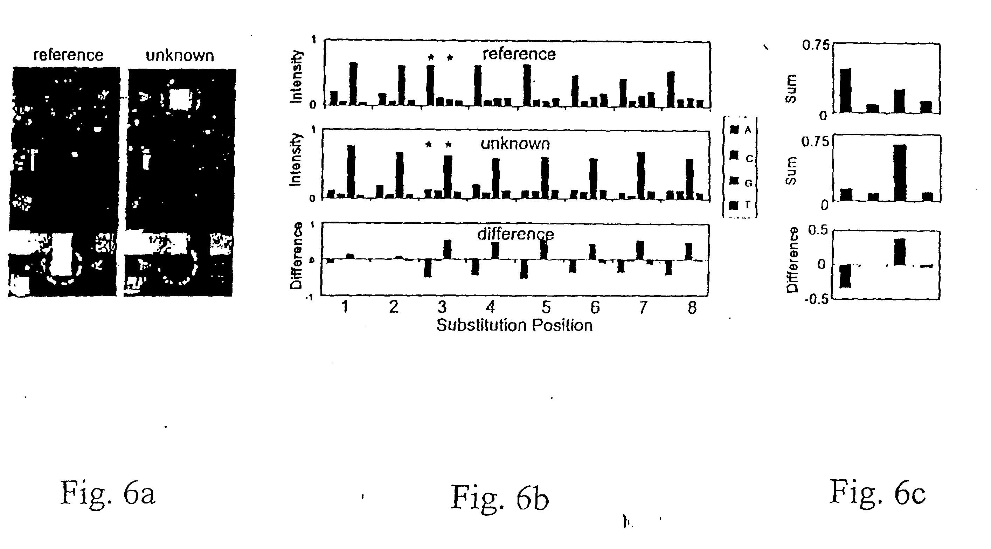

Figure 6 shows mutation discovery by comparing base call sets for DNA targets on separate arrays. The Figure illustrates an A→G substitution at position 435 of an HIV gene.

Figure 6a shows small portion of the array images for the reference (left panel) and target (right panel) are shown. The circled probes (interrogating position 435) on each array differ by a single base substitution located at the 3rd position.

Figure 6b shows the normalized intensities of the eight base call sets interrogating position 435 for both the reference and the target. The intensity differences (target relative to reference) between these base call sets are also shown.

Figure 6c illustrates the composite base call set (both strands combined) for the reference and the target and their composite difference (I_{unknown} - I_{reference} ).

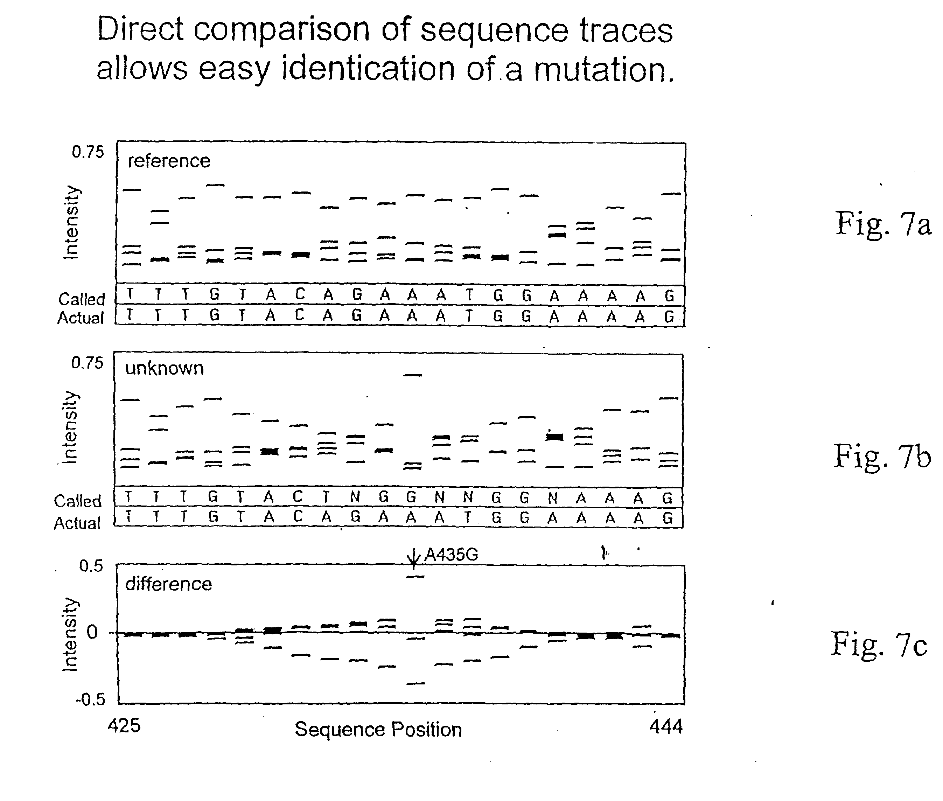

Figure 7 shows a direct comparison of sequence traces allowing easy identification of a mutation. Figures 7a and 7b show sequence traces generated from two 1.2 kb IIIV gene polynucleotides (reference and target). Figure 7c illustrates how the difference (mutation scan) in sequence traces exhibits a footprint 12-14 bases in width at the mutation site (A435G).

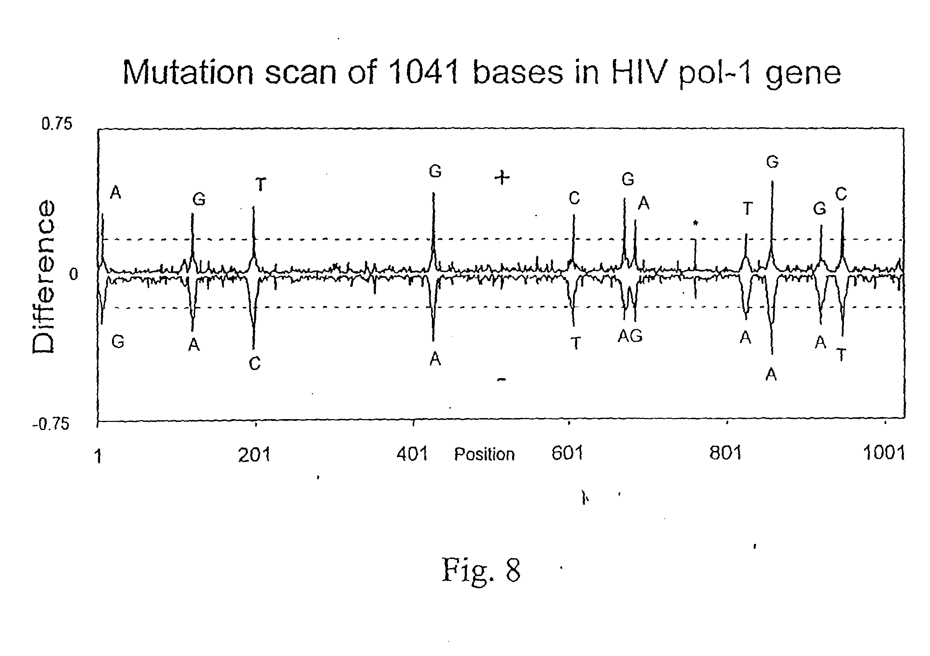

Figure 8 shows a mutation scan of 1041 bases in HIV pol-1 gene. The positive and negative envelope curves of the difference trace are plotted for 1041 bases of the 1.2 kbp HIV sequence. The Figure shows eleven single base substitutions that were correctly identified by this method. The asterisk denotes a potential mutation, but is scored negative since the peak fails to display a wide profile characteristic of mutation.

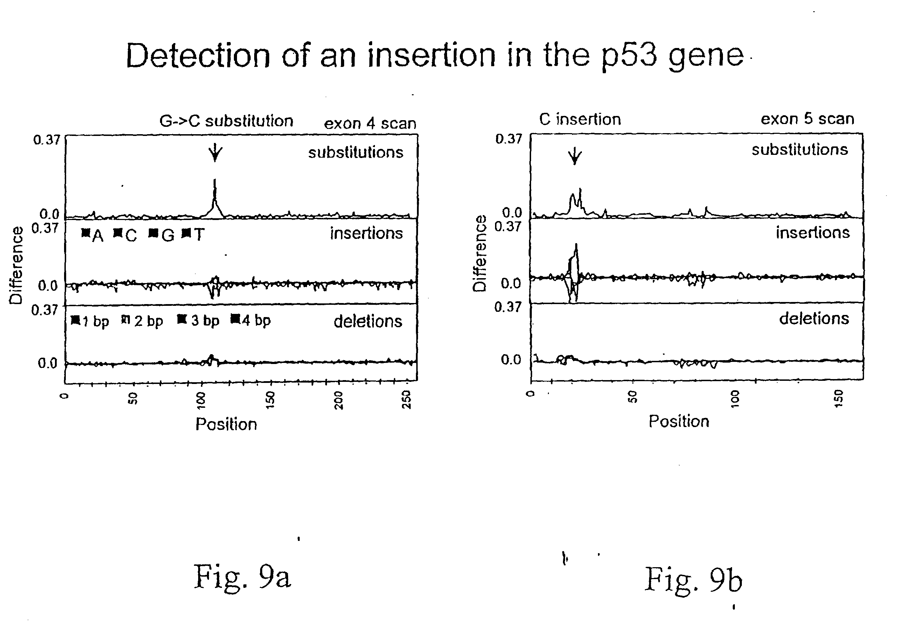

Figure 9 shows the detection of an insertion in the p53 gene. Eleven exons of the p53 gene were simultaneously scanned (~ 1700 bases including primers). Figure 9a illustrates how substitution scan (positive envelope) of exon 4 revealed a mutation footprint that correctly identified a G→C base change. Figure 9b shows that mutation scans of exon 5 exhibit footprints in both the substitution (positive envelope) and insertion scans. The insertion scan identified the mutation as a C insertion (increase in the complementary G insertion probes) in the target sample, whereas the substitution scan was ambiguous.

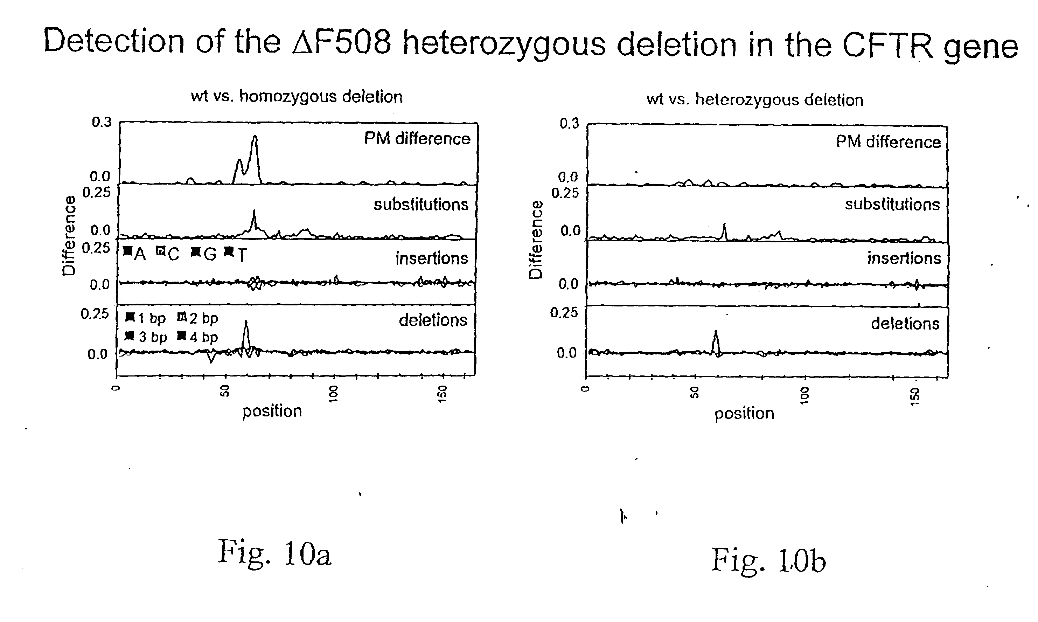

Figure 10 shows perfect match (PM) difference plot displays for homozygous and heterozygous deletions.

Figure 10a shows mutation scans of a 170 bp region comparing a wild type CFTR polynucleotide sequence to a target containing a 3-bp DF508 (TTT) homozygous deletion. Mutation scans are also shown for substitution (positive envelope only), insertion, and deletion probes. A 3-bp deletion (identified as "TTT" by the analysis software) was readily detected by the deletion scan.

Figure 10b shows mutation scans comparing a wild type CFTR target to a 3-bp ΔF508 "artificial" heterozygous deletion mutant in a 1079 bp region of the CFTR gene. No footprint was detectable in the PM difference scan since DNA with the wild-type reference sequence is present in both samples. However, the deletion scan correctly identified a 3-bp "TTT" deletion in the heterozygous sample.

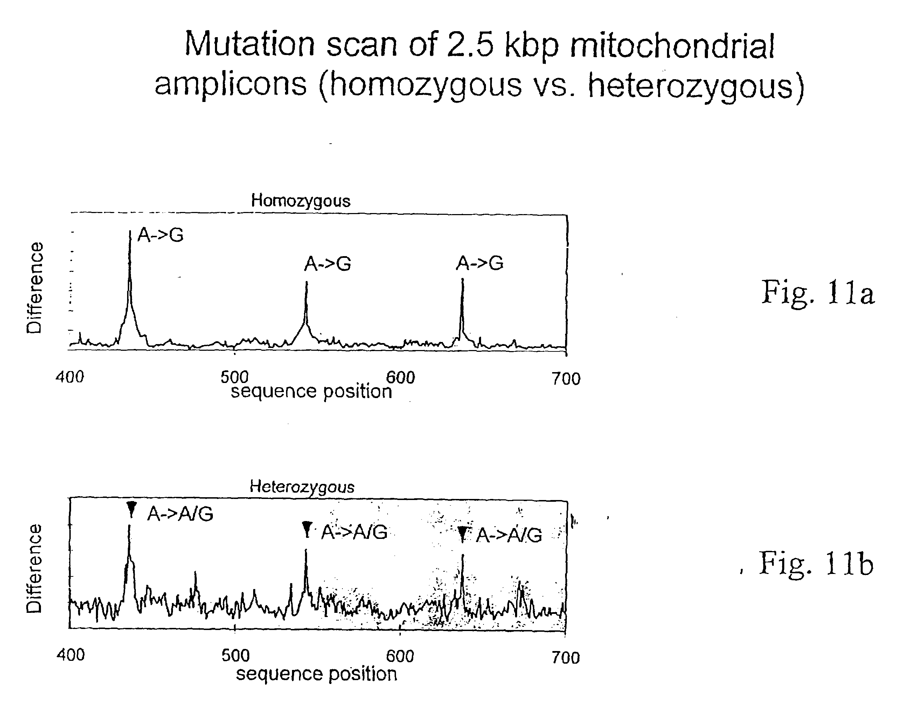

Figure 11 shows a mutation scan of 2.5 kbp mitochondrial amplicons (homozygous v. heterozygous). Figure 11a shows a 300 bp mutation scan of the homozygous sample. The scan revealed 3 single base substitutions. A total of 176 sequence differences among 8 different samples were examined. The mutation scan of the homozygous polynucleotides correctly identified over 90% of these sequence differences. Moreover, the false positive rate was quite low (< 1 per 3900 bp screened).

Figure 11b shows the same 300 bp scan as Figure 11a but the sample is a 50:50 mixture of wild type and mutated heterozygous polynucleotides. The scan revealed 3 single base substitutions, but with a decreased signal to noise ratio.

### MODES OF CARRYING OUT THE INVENTION

Throughout this disclosure, various publications, patents and published patent specifications are referenced by an identifying citation. The disclosures of these publications, patents and published patent specifications are hereby incorporated in their entirety by reference into the present disclosure.

### A. General techniques

The practice of the present invention may employ, unless otherwise indicated, conventional techniques of organic chemistry, molecular biology (including recombinant techniques), cell biology, biochemist, and immunology, which are within the skill of the art. Such conventional techniques include polymer array synthesis, hybridization, ligation, detection of hybridization using a label. Specific illustrations of suitable techniques can be had by reference to the example hereinbelow. However, other equivalent conventional procedures can, of course, also be used. Such conventional techniques can be found in standard laboratory manuals such as *Genome Analysis: A Laboratory Manual Series* (*Vols. IIV), Using Antibodies: A Laboratory Manual, Cells: A Laboratory Manual, PCR Primer: A Laboratory Manual,* and *Molecular Cloning: A Laboratory Manual* (all from Cold Spring Harbor Laboratory Press).

### B. Definitions

As used herein, certain terms may have the following defined meanings.

As used in the specification and claims, the singular forms "a", "an" and "the" include plural references unless the context clearly dictates otherwise. For example, the term "an array" may include a plurality of arrays unless the context clearly dictates otherwise.

An "array" represents an intentionally created collection of molecules which can be prepared either synthetically or biosynthetically. In particular, the term "array" herein means an intentionally created collection of polynucleotides on a support wherein the identity of each polynucleotide at a given predefined region is known. Thus, the terms "array" and "biological chips" are used interchangeably.

The array of molecules can be screened for biological activity in a variety of different formats (e.g., libraries of soluble molecules; and libraries of compounds tethered to resin beads, silica chips, or other solid supports). The fabrication of polynucleotide arrays on a solid substrate, and methods of use of the arrays in different assays, are described in: U.S. Patent Nos. 5,677,195, 5,624,711, 5,599,695, 5,445,934, 5,451,683, 5,424,186, 5,412,087, 5,405,783, 5,384,261, 5,252,743, 5,744,101, 5,143,854 and PCT WO 92/10092, the disclosures of each of which are incorporated herein.

"Solid support," "support," and "substrate" refer to a material or group of materials having a rigid or semi-rigid surface or surfaces. In many embodiments, at least one surface of the solid support will be substantially flat, although in some embodiments it may be desirable to physically separate synthesis regions for different compounds with for example, wells, raised regions, pins, etched trenches, or the like. According to other embodiments, the solid support(s) will take the form of beads, resins, gels, microspheres, or other geometric configurations.

"Predefined region" refers to a localized area on a solid support which is, was, or is intended to be used for formation of a selected molecule and is otherwise referred to herein in the alternative as a "selected" region. The predefined region may have any convenient shape, *e.g.,* circular, rectangular, elliptical, wedge-shaped, etc. For the sake of brevity herein, "predefined regions" are sometimes referred to simply as "regions." In some embodiments, a predefined region and, therefore, the area upon which each distinct compound is synthesized is smaller than about 1 cm² or even less than 1 mm². In additional embodiments, a predefined region can be achieved by physically separating the regions (*i.e.,* beads, resins, gels, etc.) into wells, trays, etc.

As used herein, a "polynucleotide" is a sequence of two or more nucleotides. Polynucleotides of the present invention include sequences of deoxyribonucleic acid (DNA) or ribonucleic acid (RNA) which may be isolated from natural sources, recombinantly produced, or artificially synthesized. A further example of a polynucleotide of the present invention may be polyamide polynucleotide (PNA). This invention also encompasses situations in which there is nontraditional base pairing such as Hoogsteen base pairing which has been identified in certain tRNA molecules and postulated to exist in a triple helix.

The term "nucleotide" includes deoxynucleotides and analogs thereof. These analogs are those molecules having some structural features in common with a naturally occurring nucleotide such that when incorporated into a polynucleotide sequence, they allow hybridization with a complementary polynucleotide in solution. Typically, these analogs may have one or modified bases, as well as modified forms of ribose and phosphodiester moieties. The changes can be tailor made to stabilize or destabilize hybrid formation or enhance the specificity of hybridization with a complementary polynucleotide sequence as desired, or to enhance stability of the polynucleotide.

Analogs also include protected and/or modified monomers as are conventionally used in polynucleotide synthesis. As one of skill in the art is well aware polynucleotide synthesis uses a variety of base-protected nucleoside derivatives in which one or more of the nitrogens of the purine and pyrimidine moiety are protected by groups such as dimethoxytrityl, benzyl, tert-butyl, isobutyl and the like.

For instance, structural groups are optionally added to the ribose or base of a nucleoside for incorporation into a polynucleotide, such as a methyl, propyl or allyl group at the 2'-O position on the ribose, or a fluoro group which substitutes for the 2'-O group, or a bromo group on the ribonucleoside base. 2'-O-methyloligoribonucleotides (2'-O-MeORNs) have a higher affinity for complementary polynucleotides (especially RNA) than their unmodified counterparts. 2'-O-MeORNA phosphoramidite monomers are available commercially, *e.g.,* from Chem Genes Corp. or Glen Research, Inc. Alternatively, deazapurines and deazapyrimidines in which one or more N atoms of the purine or pyrimidine heterocyclic ring are replaced by C atoms can also be used.

The phosphodiester linkage, or "sugar-phosphate backbone" of the polynucleotide can also be substituted or modified, for instance with methyl phosphonates, O-methyl phosphates or phosphorothioates. Another example of a polynucleotide comprising such modified linkages for purposes of this disclosure includes "peptide polynucleotides" in which a polyamide backbone is attached to polynucleotide bases, or modified polynucleotide bases. Peptide polynucleotides which comprise a polyamide backbone and the bases found in naturally occurring nucleotides are commercially available from, *e.g.,* Biosearch*,* Inc. (Bedford, MA). See also U.S. Patents Nos. 5,773,571 and 5,786,461.

Nucleotides with modified bases can also be used in this invention. Some examples of base modifications include 2-aminoadenine, 5-methylcytosine, 5-(propyn-1-yl)cytosine, 5-(propyn-1-yl)uracil, 5-bromouracil, 5-bromocytosine, hydroxymethylcytosine, methyluracil, hydroxymethyluracil, and dihydroxypentyluracil which can be incorporated into polynucleotides in order to modify binding affinity for complementary polynucleotides.

Groups can also be linked to various positions on the nucleoside sugar ring or on the purine or pyrimidine rings which may stabilize the duplex by electrostatic interactions with the negatively charged phosphate backbone, or through interactions in the major and minor groves. For example, adenosine and guanosine nucleotides can be substituted at the N² position with an imidazolyl propyl group, increasing duplex stability. Universal base analogues such as 3-nitropyrrole and 5-nitroindole can also be included. A variety of modified polynucleotides suitable for use in this invention are described in, *e.g.,* "Antisense Research and Application", S.T. Crooke and B. LeBleu (eds.) (CRC Press, 1993) and "Carbohydrate Modifications in Antisense Research" in ACS Symp. Ser. #580, Y.S. Sanghvi and P.D. Cook (eds.) ACS, Washington, D.C. 1994

A "target" polynucleotide is a polynucleotide to be characterized, for example, by its mutations. In general the sequence of a target polynucleotide is not known. However, in some aspects, for example, when determining whether a plurality of target polynucleotides are identical, the sequence of one of the polynucleotides may be known. A target can be derived from any biological source and even can be partially or fully synthesized.

A "reference" polynucleotide is a polynucleotide whose hybridization pattern to a probe array is to be compared with the hybridization pattern of a target polynucleotide to another identical or substantially identical probe array. The sequence of a reference polynucleotide may be known but it is not essential that the sequence be known.

"Fragments" of a polynucleotide are portions of the polynucleotide that may be of any length. Such fragments may be single-stranded or double-stranded polynucleotides, or may contain both single- and double-stranded portions.

A "polynucleotide probe" is any arrangement of single- and double-stranded polynucleotides that can be used in the hybridization and ligation steps described herein. Most commonly, a polynucleotide probe will consist of a double-stranded polynucleotide region covalently attached to a single-stranded polynucleotide region. The polynucleotide sequence for the double stranded region in each probe may be identical or different. For ease of manufacture, the double stranded region may be identical in each probe and such identical region may also be known as "constant" region.

The nucleotides in the single stranded region are preferably variable. This single-stranded variable region is known as an "overhang" and this overhang can be 5' or 3' oriented, i.e., can possess a free 5'- end or a free 3'- end, respectively. Further the overhang can be of several nucleotides (an "n-mer"), ranging, for example, from about 4 to about 50 nucleotides. Preferably, the overhang is from about 5 to about 20 nucleotides, more preferably from about 6 to about 12 nucleotides, and most preferably from 8 to 9 nucleotides.

The term "hybridization" refers to the process in which two single-stranded polynucleotides bind non-covalently to form a stable double-stranded polynucleotide; triple-stranded hybridization is also theoretically possible. The resulting (usually) double-stranded polynucleotide is a "hybrid." The proportion of the population of polynucleotides that forms stable hybrids is referred to herein as the "degree of hybridization."

Methods for conducting polynucleotide hybridization assays have been well developed in the art. Hybridization assay procedures and conditions will vary depending on the application and are selected in accordance with the general binding methods known including those referred to in: Maniatis et al., "Molecular Cloning: A Laboratory Manual" 2nd Ed., Cold Spring Harbor, N.Y., 1989; Berger and Kimmel, "Methods in Enzymology," Vol. 152, "Guide to Molecular Cloning Techniques", Academic Press, Inc., San Diego, CA., 1987; Young and Davis, *Proc. Natl. Acad. Sci.,* U.S.A., 80: 1194 (1983), each of which are incorporated herein by reference.

It is appreciated that the ability of two single stranded polynucleotides to hybridize will depend upon factors such as their degree of complementarity as well as the stringency of the hybridization reaction conditions.

As used herein, "stringency" refers to the conditions of a hybridization reaction that influence the degree to which polynucleotides hybridize. Stringent conditions can be selected that allow polynucleotide duplexes to be distinguished based on their degree of mismatch. High stringency is correlated with a lower probability for the formation of a duplex containing mismatched bases. Thus, the higher the stringency, the greater the probability that two single-stranded polynucleotides, capable of forming a mismatched duplex, will remain single-stranded. Conversely, at lower stringency, the probability of formation of a mismatched duplex is increased.

The appropriate stringency that will allow selection of a perfectly-matched duplex, compared to a duplex containing one or more mismatches (or that will allow selection of a particular mismatched duplex compared to a duplex with a higher degree of mismatch) is generally determined empirically. Means for adjusting the stringency of a hybridization reaction are well-known to those of skill in the art. See, for example, Sambrook, *et al.,* "Molecular Cloning: A Laboratory Manual," Second Edition, Cold Spring Harbor Laboratory Press, 1989; Ausubel, *et al.,* "Current Protocols In Molecular Biology," John Wiley & Sons, 1987, 1988, 1989, 1990, 1991, 1992, 1993,1994, 1995, 1996 and periodic updates; and Hames *et al.,* "Nucleic Acid Hybridization: A Practical Approach," IRL Press, Ltd., 1985.

In general, conditions that increase stringency (*i.e.*, select for the formation of more closely-matched duplexes) include higher temperature, lower ionic strength and presence or absence of solvents; lower stringency is favored by lower temperature, higher ionic strength, and lower or higher concentrations of solvents (for example, lower concentrations of formamide or dimethyl sulfoxide). The duration of the hybridization reaction and the concentration of reactants (*i.e.*, single stranded polynucleotide) can also affect stringency, with short reaction times and low reactant concentrations favoring higher stringency.

The term "ligation" refers to the process of covalently linking two terminal nucleotides that are on two separate polynucleotide strands, whereby the two strands are joined into one single strand. In the present methodology, in some aspects, hybridization and ligation are accomplished in one step.

An "n-mer" is a single-stranded polynucleotide of "n" number of nucleotides. A "complete set" of n-mers refers to a set of single-stranded polynucleotides of "n" number of nucleotides wherein the set represents every possible combination of the "n" nucleotides. For example, when "n" represents 8, a complete n-mer set represents 4⁸ single-stranded polynucleotides. A "substantially complete set" of n-mers represents a set that may not have every possible combination of the "n" nucleotides but provides sufficient number of such single strands to be used as probes such that mutations in a target polynucleotide can be characterized. Thus, in some aspects, such a substantially complete set may contain a subset of the single-stranded polynucleotide probes.

The term "substantially identical" as used in the context of array description refers to arrays that may not be identical with respect to each probe composition. However, such arrays are functional with respect to participating in the hybridization and ligation steps of the present invention such that mutations in a target polynucleotide may be characterized. For example, two arrays may be substantially identical if one array provides a probe with a certain base at a certain location and the other array provides an analog of that base at the corresponding location.

The term "base call" refers to the determination of the identity of an unknown base in a target polynucleotide. Base-calling is made by comparing the degree of hybridization between the target polynucleotide and a probe polynucleotide with the degree of hybridization between a reference polynucleotide and the probe polynucleotide. In a given case a base call can be made or withheld by selecting a threshold degree of hybridization.

A "base call set" is the set of populations of polynucleotides which will yield a base call for a particular nucleotide or a sequence of nucleotides in a target polynucleotide. Thus, each base call set consists of four probes differing at single substitution position (indicated by the letter N in Figure 3), located so as to interrogate the specified target base.

"Mutation" is a change in the nucleotide sequence of a polynucleotide from that of the normal ("wild-type") population. A "substitution" mutation is one in which a nucleotide is replaced by another nucleotide. A "deletion" mutation is one in which one or more nucleotides are deleted from the normal sequence, and an "insertion" mutation is one in which one or more nucleotides are inserted into the normal sequence. A "homozygous" mutation is one that appears in both chromosomes in a diploid organism. A "heterozygous" mutation is one that appears in only one chromosome in a diploid organism, the other chromosome being normal. A mutation can be characterized as, for example, an A→G substitution at position 435.

"Polymorphism" is the occurrence in the same population of multiple discrete allelic states of which at least one has high frequency (conventionally > 1% or more). One of ordinary skill in the art knows that the term "polymorphism" is related to mutation.

Throughout this disclosure, various aspects of this invention are presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, the description of a range such as 4 to 50 should be considered to have specifically disclosed all subranges such as 4 to 10, 4 to 20,4 to 30,4 to 40, 4 to 50, 5 to 10, 5 to 20 etc., as well as individual numbers within that range, for example, 6, 8, 15, 20, 32, 39, 43, 48 etc. This applies regardless of the breadth of the range.

### C. The Methods

### 1. Overview

The invention provides methods to determine the presence of mutations in a target polynucleotide using complete or substantially complete n-mer arrays of polynucleotide probes having double-stranded polynucleotide regions with variable single-stranded regions known as "n-mer overhangs." One method comprises hybridizing fragments of a target polynucleotide with the polynucleotide probes of the array. The polynucleotide probes of another identical or substantially identical array are similarly hybridized with fragments of a reference polynucleotide. The patterns of hybridization of the target and reference polynucleotides on the arrays can be directly compared, allowing the determination of the presence of mutations.

Sensitivity and accuracy of the methodology can be increased by generating "virtual tilings" (see below) from the hybridization patterns and comparing the vinual tilings directly. Such direct comparison subtracts out potentially confusing signals resulting from systematic and reproducible cross-hybridization and multiple probe-target interactions. The methods afforded greater than 90% mutation detection sensitivity in 2.5 kbp targets (on 8-mer arrays), with extremely high accuracy (*i.e.,* low number of false positives, <0.02% per base). The virtual tiling step permits focused search for the detection of unanticipated mutations (insertions, deletions, multiple point mutations) within a target by electronically assembling the appropriate query probes.

As disclosed herein, the present methods involve not only the determination of the presence of a mutation but also the identification of the location and type of the mutation, *i.e.*, whether the mutation is a single or multiple base substitution, deletion or insertion. The methods can also be used to detect both homozygous and heterozygous mutations as well as polymorphisms. Further, the methods can be used to compare two or more unknown polynucleotide sequences to determine if they are identical, without necessarily having to know the sequence of one of the polynucleotides to act as a reference. These methods permit such analyses because, unlike the traditional techniques which compare sequences, these methods compare hybridization patterns.

Thus, multiple polynucleotides can be rapidly and accurately compared to determine if they are identical in sequence. These rapid and accurate methods are particularly useful for forensic purposes to compare polynucleotides from a variety of sources without necessarily sequencing or having to know the sequence of any of the polynucleotides. These methods may offer advantages over the conventional methods in terms of their accuracy, sensitivity, and thus can be expected to gain wide-spread acceptance in a court room. Further, the methods can be used to screen quickly the mutational effects of drugs, chemicals, radiation, stress or environmental factors on genes, by comparing the hybridization patterns before and after exposure. The versatility of the methods can be further exploited by using parallel processing techniques described below and in the U.S. Patent No. 5,545,531, whereby multiple polynucleotide sequences can be analyzed simultaneously.

The use of standardized, complete or substantially complete arrays obviates the need to construct customized arrays for each polynucleotide to be analyzed, providing a "one-size-fits-all" methodology that can be used for virtually any polynucleotide analysis. Thus, the methods provide a general framework for the development of techniques alternative to the traditional gel-based analysis of complex polynucleotide samples and methods based entirely on sequence determination.

One method of practicing the invention involves the use of identical or substantially identical probe arrays for hybridizing with reference and target polynucleotides. The invention can also be practiced with a single array to which reference and target polynucleotides are successively hybridized, with the pattern of hybridization of each analyzed and stored. Alternatively, two sets of probes can be provided on one substrate, wherein the two probe sets are distinctly colored or labelled such that the invention can be practiced for both the target and the reference simultaneously.

The methods described herein contemplate the use of two or more arrays, individually, wherein each array constitutes a complete n-mer set of probe polynucleotides. It is entirely within the ordinary skill of the art to perform the methods (hybridizations, ligations and characterizations) in parallel, wherein multiple arrays can be provided on a plate (a biological chip plate) having a plurality of test wells, and each test well includes a biological chip or array. Such parallel methods of analysis are described in the U.S. patent No. 5,545,531, which is herein incorporated by reference in its entirety.

Briefly, the target and reference polynucleotides can be introduced into the test wells. A fluid handling device may expose the test wells to a chosen set of reaction conditions by, for example, adding or removing fluid from the wells, maintaining the liquid in the wells at predetermined temperatures, and agitating the wells as required, thereby performing the various hybridization, ligation and washing steps. Then, an array reader can interrogate the probe arrays in the test wells, thereby obtaining the hybridization data. A computer having an appropriate program can further analyze the hybridization patterns as described herein. Such parallel processing greatly improves efficiency.

The present methods can be used to characterize mutations associated with any polynucleotide. For example, mutations associated with cystic fibrosis, including mutations in the CFTR gene, tumor suppressor p53 gene, and the HIV-1 pol gene can be characterized. The methods also provide techniques for detecting specific sequences of mitochondrial DNA for forensic identification purposes such as DNA finger-printing, sequence-specific 5'-end sampling, and techniques useful for differential gene expression assays, enzymatic and chemical mismatch cleavage, and polynucleotide scanning.

### 2. Complete or Substantially Complete n-mer Arrays

### a) Array Characteristics

An array of polynucleotide probes of this invention comprises polynucleotide probes attached to a substrate at predefined regions. One example of a polynucleotide probe is shown in Figure 1.

Each probe comprises a double-stranded region and a single-stranded region. The double-stranded portion of the probes are, in one aspect, linked covalently to a solid support. The double-stranded portion is optionally covalently crosslinked using a cross-linking agent such as psoralen. The double-stranded portion of the probe can be from about 4 to about 200 or more nucleotides long. In one aspect, the double-stranded portions of the polynucleotide probes consist of a nucleotide sequence that is constant, *i.e.,* does not vary from one probe to the other in a given array. It is however possible, and may be desirable in some cases, to practice the invention using double-stranded portions which are variable from probe-to probe. The presence of the double-stranded region allows a ligation step to be performed after the hybridization step in the method.

The single-stranded regions of the polynucleotide probes are known as "overhangs" and these overhangs can be 5'- or 3'-oriented, *i.e.,* can have a free 5'- end or a free 3'- end, respectively. Figure 1 shows an overhang that is 5'-oriented. The overhang may be of any length ("n") of diverse nucleotides, known as an "n-mer." A complete "n-mer array" as described herein contains every possible combination of sequences of length "n" in the single-stranded portions of the probes. Since any of the four bases can be present at any position in the overhang, the total number of possible nucleotide sequences for the single-stranded portions of the probes of a complete array is 4ⁿ. For example, if n = 8, the total number of probe sequences will be 4⁸ = 65,536. The length of the overhang can be, for example, from about 4 to about 50 or more nucleotides, although shorter and longer strands can also be used. Preferably, the overhang is from about 6 to about 12 nucleotides, and more preferably, from about 8 to about 9 nucleotides.

Though complete arrays are preferred, it is not necessary for the practice of the invention that the arrays be complete. The arrays can be substantially complete and the invention may still be practiced. A "substantially complete set" of n-mers represents a set that may not have every possible sequence "n" nucleotides, but provides a sufficient number of different sequences to be used as probes for characterizing mutations in a target polynucleotide.

### b) Array Synthesis

Complete or substantially complete arrays of polynucleotide probes can be synthesized according to art-known methods. A linker, such as DMT-hexaethyleneglycol-(2-cyanoethyl-N,N-diisopropyl) phosphoramidite, may be attached to a derivatized solid support, such as a glass or silica support. All but the last of the nucleotides of a single strand of the double-stranded anchor region are synthesized using conventional chemical synthesis, for example using DMT PAC phosphoramidites. Such techniques are well-known in the art. See, for example, U.S. Patent Nos. 4,458,066, 4,500,707, 4,725,677, and Jones, chapter 2, and Atkinson et al, chapter 3, in Gatl, ed., *Oligonucleolide Synthesis: A Practical Approach* (IRL Press, Washington, D.C., 1984); Froehler et al, *Tetrahedron Letters,* 27: 469-472 (1986)

The last nucleotide of that single strand of the double-stranded region, the strand complementary to that single strand, along with the "n" nucleotides of what will become the single-stranded n-mer overhang, can be synthesized, for example, by "light-directed" photolithographic techniques. It is understood that on the probe, one strand is longer than the other, such that the two strands are complementary for the entire length of the shorter strand (thus forming the double stranded portion of the probe), and the overhang is that portion of the longer strand that extends beyond the hybridized portion. See Figure 1.

Light-directed nucleic acid synthesis techniques are well-known in the art. See, for example, U.S. Patents Nos. 5,143,854, 5,424,186, 5,445,934, 5,527,681, 5,677,195, and 5,770,722, the disclosures of which are incorporated herein.

Briefly, in the light-directed techniques, selected regions of the substrate are exposed to light to activate those regions, and nucleotide monomers with photoremovable groups are attached to the activated regions. The monomer can be a phosphoramidite activated nucleoside protected at the 5'-hydroxyl with a photolabile protecting group. The steps of activation and attachment are repeated until polynucleotides of the desired length and sequences are synthesized. Photolabile groups are then optionally removed and the sequence is, thereafter, optionally capped. Side chain protective groups, if present, are also removed.

These photolithographic and fabrication techniques enable each probe sequence to occupy a very small area on the support. For example, a probe array of 0.25 mm² could have from a 1000 to 10⁴ or 10⁵ or 10⁶ features. Further details on achieving the desired probe density can be found in U.S. Patent No. 5,510,270, the disclosure of which is incorporated by reference.

Computer tools may be used for forming arrays using the photolithographic techniques. For example, a computer system may be used to select polynucleotide or other polymer probes on the substrate, and design the layout of the array. See U.S. Patent No. 5,571,639, the disclosure of which is incorporated herein. Alternatively, the array can be synthesized using the mechanical techniques disclosed in U.S. Patent No. 5,384,261, incorporated herein by reference.

The substrate is preferably flat but may take on a variety of alternative surface configurations. The substrate and its surface are also chosen to provide appropriate light-absorbing characteristics. For instance, the substrate may be functionalized glass, Si, Ge, GaAs, GaP, SiO₂, SiN₄, modified silicon, or any one of a wide variety of gels or polymers such as (poly)tetrafluoroethylene, (poly)vinylidenedifluoride, polystyrene, polycarbonate, polypropylene, or combinations thereof. Other substrate materials will be readily apparent to those of skill in the art upon review of this disclosure. in a preferred embodiment the substrate is flat glass or silica.

Various photocleavable protecting groups and methods for their use are known in the art. See for example, U.S. Patent Nos. 5,489,678, 5,744,101 and 5,753,788. The protecting groups are typically removed by photolysis, *i.e.,* by irradiation at wavelengths greater than about 340 nm, preferably at about 365 nm. The photolysis is usually conducted in the presence of hydroxylic or protic solvents, such as aqueous, alcoholic or mixed aqueous-alcoholic or mixed aqueous-organic solvent mixtures. Alcoholic solvents frequently used include methanol and ethanol. Photolysis is frequently conducted at neutral or basic pH. Alternatively, it may be advantageous to use acid or base catalyzed cleavage conditions. Such chemical cleavage methods are described, for example, in the U.S. Patent No. 5,599,695.

In some aspects, following the synthesis of the double-stranded region and the n-mer overhang region, two bases of inosine can be added to, for example, the 5' terminus, using conventional DMT phosphoramidite chemistry. The addition of two inosines improves overall array signal intensities without greatly affecting discrimination.

The anchor double-stranded polynucleotide may contain appropriate base combinations and/or chemical constituents to allow for optional covalent cross-linking, such as an AT dinucleotide pair and a psoralen C6 moiety at the 5' end. In general, anchor double-stranded polynucleotide portions of the probes with A and G (purines, preferably G) 3'-bases generate higher signal intensities than polynucleotides with C and T (pyrimidines) 3'-bases. The single-stranded n-mer is then contacted with the array, and will hybridize with the complementary anchor sequence to form a double-stranded portion with a single-stranded overhang. The orientation of the overhang can be changed to a 3' or 5' as desired. By this, it is meant that the overhang can have a free 3'-, or 5'-end. A 3'-hydroxyl end is preferred for enzymatic ligation whereas a 3' phosphoryl version is preferred for chemical ligation.

### 3. Fragmenting, Hybridizing and Ligating

Target and or reference polynucleotides are fragmented, and, if necessary, denatured, to single-stranded fragments. The fragments can be of variable length, random or quasi-random, preferably not shorter than the n-mer overhangs. In some aspects, the fragments can be upto about 100 base pairs or longer, while fragments of from about 20 to about 60 base pairs may be preferred. One of ordinary skill in the art would understand that the fragment size can be easily optimized as desired. The fragmentation can be accomplished by chemical, enzymatic, or other means known in the art. In one aspect, a nuclease, for example, a DNase I if the target polynucleotide is a DNA, can be used.

Following fragmentation, the polynucleotide, if double-stranded, is denatured, for example by heating at 95 °C for 15 min. By this procedure, a polynucleotide is reduced to a random or quasi-random population of phosphoryl terminal single-stranded subsequences representative of its entire sequence. While the phosphoryl terminal single-stranded subsequences may be 3'- or 5'- oriented, it is preferable that they are 5'-oriented. Both the complementary strands will be represented. Optionally, the fragments may be end-labeled (either on their 3'- or on their 5'- ends) with end labeling agents known in the art, such as terminal dideoxynucleotidyl transferase and ddATP-N6-biotin, or polynucleotide kinase and a phosphorous-labeled ATP.

Hybridization of the fragmented reference and target polynucleotides to the probe overhangs can be performed under conditions that are well-known in the art. It is also well-known that hybridization under conditions of optimal stringency decreases mismatches. Means for adjusting the stringency of a hybridization reaction are well-known to those of skill in the art. See, for example, Sambrook, et al., "Molecular Cloning: A Laboratory Manual," Second Edition, Cold Spring Harbor Laboratory Press, 1989; Ausubel, et al., "Current Protocols In Molecular Biology," John Wiley & Sons, 1996 and periodic updates; and Hames et al., "Nucleic Acid Hybridization: A Practical Approach," IRL Press, Ltd., 1985.

In one aspect, hybridization can be performed at a pH of from about 7.0 to about 8.0, preferably, about 7.4, in a buffer such as a 6X SSPE buffer, at a temperature of from about 25° C to 45° C, preferably, at about 40° C, and for about 1-2 hr. In another aspect, the hybridizations of the present invention can be performed using betaine as a signal enhancer. The use of betaine to improve hybridization signal is described in the copending application, U.S. Serial No. 08/648,709, filed May 16, 1996 (now allowed). Subsequent to hybridization, the array is washed, for example, on a GeneChip^{TM} Fluidics Station (from Affymetrix, Inc.) with 6X SSPE at 22 °C, in preparation for ligation.

The "n" nucleotides at the appropriate terminus of every fragment will find an exactly complementary n-mer on the single-stranded portion of a probe with which to hybridize, if the array is complete or substantially complete. Although the fragment may be longer than "n" nucleotides, the information to be gained from the hybridization will come from the "n" nucleotides of the fragment that hybridize with their exactly complementary sequence on the probe. Hence, every terminal nucleotide sequence in the target polynucleotide of length "n" is represented by a hybridized probe in the array.

The method may also comprise the additional step of ligation to the probe, whereas in some aspects, both hybridization and ligation can be accomplished in a single step. It is understood that the term "ligation to the probe" refers to the process wherein the hybridized target or reference polynucleotide fragment is ligated to the shorter strand of the probe. See Figure 1. Only the terminal mucleotides of the strands arc ligatable. It is further understood that the target and reference fragments are hybridized separately to different but identical or substantially identical arrays. When the method is used to analyze two or more unknown polynucleotides, the fragments of each unknown polynucleotide is hybridized separately to a different but identical or substantially identical array.

Ligation allows thorough washing of the array to remove non-hybridized polynucleotide fragments. Thus, in one aspect, the fragmented and denatured polynucleotides are cooled on ice, added to the ligation mix, spun in a microfuge to pellet any precipitate present, and applied to the polynucleotide probe arrays.

Both enzymatic and chemical ligation produce satisfactory results. The enzymatic ligation may use DNA ligases such as T4 DNA ligase, *E. coli* (NAD-dependent) DNA ligase, or Taq DNA ligase. Appropriate standard ligation buffer and temperature for each of these enzymes are well-known in the art. The ligation mixture is typically incubated overnight (14-16 hours), but incubations as short as 2 hours may be sufficient.

The chemical ligation reaction may employ a carbodiimide such as 1-ethyl-3-(3-dimethylaminopropyl)carbodiimide hydrochloride, to generate a pyrophosphate bond between the phosphoryl moiety of the fragments and the phosphoryl moiety on the anchor polynucleotide probe. For example, the phosphoryl moiety of the fragment may be 5'-oriented while the phosphoryl moiety of the anchor probe is 3'-oriented. The chemical ligation typically proceeds for 14 hrs at 30 °C. In one aspect, up to 3 to 4 M tetramethylammonium chloride (TMACl) may be used in the ligation buffer in order to help normalize the stability of A/T-rich and G/C-rich hybrids.

Following ligation, arrays are washed, for example, on a GeneChip^{TM} fluidics station as described above. Optionally, the array may be stained with any art-known stain and then washed. One example of such stain is streptavidin-phycoerythrin conjugate.

### 4. Determining the Presence of Mutations

### a) Hybridization Pattern

Any method by which the hybridization pattern may be assessed can be used. Several such methods are known in the art. See for example, US Patent No. 5,599,695. In one preferred method, the fragments of the target and reference polynucleotides are labeled. In a more preferable aspect, the label is a fluorescent label, obtained by biotin-labeling followed by streptavidin-phycoerythrin conjugate formation. The fluorescent signal from the array may be detected using specially designed confocal scanners which, for example, image 8-mer arrays at 6.8-7.5 µm resolution and image 9-mer arrays at 3.5 µm resolution. Images are processed with, for example, GeneChip^{TM} software (Affymetrix, Inc., Santa Clara, CA), and the resultant intensity data can be transformed into a text file containing an alphabetized list of probe sequences and corresponding intensities. These data are then analyzed by custom software written in, for example, Visual Basic^{TM} (v. 4.0, Microsoft).

The fluorescent intensities can be background subtracted and then normalized by dividing the intensity of each probe (all 4ⁿ probes) by the sum of the intensities of all related single base mismatch (MM) probes (24 MM probes for every probe on an 8-mer array) and the intensity of the probe itself. The formula is: Iₙₒᵣₘ = I/(Σ I_{MM} + I). This normalization algorithm improves base calling accuracy by moderating the sequence-dependent intensity variation of the probes (see below for description of base calling). Normalization is also performed at the level of the base call sets such that the sum of intensities of the four probes within a base call set are always unity. This prevents any one base call set from dominating the composite average.

### b) Base calling

"Base calling" is the process of identifying the base in a nucleotide at a particular position in a nucleotide sequence. As stated above, the "n" nucleotides at the terminus of each target or reference polynucleotide fragment will hybridize with an exactly complementary n-mer overhang. In addition, for a given base in the n nucleotides at the terminus of the fragment, there will also exist three probes on the array that differ from the exactly complementary probe only in the identity of that one base. For example, if the base is adenine, its complementary base would be thymine, and the three other probes which are not complementary would contain at that position adenine, guanine, or cytosine. Hence, for a given base in a "n"-length subsequence, there exists one perfect complementary probe and three probes that are non-complementary, differing only in that one base. This set of probes is called a "base-call" set. See U.S. Pat. No. 5,837,832, herein incorporated by reference.

Each nucleotide in a polynucleotide sample will give rise to "p" number of base call sets, where "p" represents the number of nucleotides in the polynucleotide. See Figure 3a for an example of a base call set. If both the complementary strands are analyzed, the number of base call sets for each nucleotide increases to 2p. Thus each base is queried redundantly, increasing the accuracy of the comparison of samples. In a given four-nucleotide base call set, the degree of hybridization will be greatest for the probe which is exactly complementary to the nucleotide sequence in the polynucleotide sample. See the illustration in Figure 3b. Because of the redundancy of the base call sets for a given nucleotide, summing the degree of hybridization across all base call sets for a nucleotide gives a highly accurate method of identifying the nucleotide. This is illustrated in Figure 3c. This reasoning can be extended to nucleotides other than the usual A, T, G, and C, for example to nucleotide analogs.

The information thus obtained can be displayed for consecutive nucleotide positions in a "trace," which shows the most likely base call for each position in a sequence. See Figure 4. A sequencing trace is constructed by plotting the intensity values for each member of a base call set as a function of sequence position. In Figure 4, positions 66-68 are plotted for the cystic fibrosis gene fragment of 540 base pairs. The intensities are normalized such that the four probe intensities within each base call set sum to one.

In most cases, the base call is clear from a comparison of the degree of hybridization to the different members of a base call set, but in some cases the differences in degree of hybridization are not great enough to make a call. The cutoff point for a call or no-call may be found through the use of a confidence score. The confidence score (quality) of the base call is defined as follows: Iₘₒₛₜ₋ᵢₙₜₑₙₛₑ/Iₙₑₓₜ₋ₘₒₛₜ₋ᵢₙₜₑₙₛₑ for probes within a base call set. If this score is below a threshold of 1.15, the base is classified as a no call. Using this measure, most incorrect calls were characterized by low confidence scores. See Figure 5b.

### c) Virtual Tiling

Probes exactly complementary to a given mutation will hybridize to a greater degree to fragments from a target polynucleotide containing that mutation than they will to the reference sequence. These probes can be selected for further analysis. This strategy of selecting the appropriate probes for examining mutations in a given polynucleotide is called "virtual tiling," a concept similar to physical tilings that are actually present on the probes in the arrays. The basics of tiling are further explained in Chee *et al., Science, 274:* 610-14 (1996), which is incorporated herein by reference.

In physical tiling, the array of probes is constructed to contain sets of probes corresponding only to mutations in a specific gene, *i.e.,* the interrogation probes are pre-chosen prior to synthesis on the array based on the specific nucleotide sequence to be analyzed. In contrast, virtual tiling involves "electronically" assembling the appropriate interrogation probes from the complete n-mer probe set. This design creates multiple probe interrogations for every target base, generating redundant base call sets that can be integrated through a simple normalized summation (averaging) process. Thus, every base in a polynucleotide sequence can be redundantly probed by "n" base-call sets electronically extracted from the complete or substantially complete polynucleotide probe array.

In addition, virtual tiling allows probe sets to be constructed to test for deletions or insertions in the target polynucleotide compared to the reference polynucleotide, and with a high degree of accuracy. Such direct comparison subtracts out potentially confusing signals resulting from systematic and reproducible cross-hybridization and multiple probe-target interactions. Thus, using complete or substantially complete n-mer arrays and the method of virtual tiling, one can characterize mutations for virtually any polynucleotide without having to construct arrays customized for that polynucleotide. Further, the information extracted can also be used for primary sequencing-by-hybridization on polynucleotides, such as genomic DNA fragments or to assess the accuracy of sequencing achieved by other means.

### d) Determining the Presence of Mutations

While the present invention may be used for sequencing purposes, in its most powerful form, the invention does not require determination of the sequence of the target polynucleotide, nor does it necessitate that the sequence of the reference polynucleotide be known. Rather, the hybridization patterns of the reference and the target polynucleotides are directly compared. From this comparison one may determine the mutations in the target polynucleotide with a high degree of accuracy without sequencing the target polynucleotide.

### (i) Characterizing Substitution Mutations

To compare the reference and the target polynucleotides for substitution mutations, one extracts (preferably electronically) from each array of probes the group of probes that represent the complete group of base call sets for the known sequence of the reference sample. For each member of each base call set, the degree of hybridization of target polynucleotide fragments, as represented by the intensity of label, is compared to the degree of hybridization of the reference fragments by taking the arithmetic difference. If the base at a given position in the sequence is the same in both the target and the reference, the difference in degree of hybridization for all four bases in a base call set will be approximately zero.

However, if the target polynucleotide contains a substitution mutation at that base, a difference in degree of hybridization is seen for two of the four members of a base call set, representing the probe complementary to the fragment containing the original base and the probe complementary to the fragment containing the base present in the mutation. See Figure 6, which illustrates the characterization of an A→G substitution at position 435 in the HIV gene. This difference in two members of the base call set will be seen, in all "p" base call sets which hybridize to subsequences containing the mutation (2n if both forward and reverse strands are analyzed). The presence of a mutation in a target is easily visualized as a broad (10-14 base) footprint encompassing the mutation site. See the difference in mutation scan in sequence traces as shown in Figure 7c. This footprint results from a single base change affecting overlapping probes upstream and downstream of the mutation site (when both strands are used). The methodology is further discussed in Example 2 below.

A further visual simplification can be realized by plotting just the positive and negative envelopes of the difference trace and noting the corresponding bases creating the relevant maximum positive score and maximum negative score. This type of plot is referred to as a "substitution" mutation scan, which is illustrated in Figure 8. The Figure clearly shows eleven single base substitutions that were correctly identified by this method. The identified substitutions were: G14A, A128G, C206T, A435G, T614C, A679G, G693a, A832T, A865G, A927G, T955C.

### (ii) Characterizing Insertion and Deletion Mutations

Using the above-described principles, base call sets to test for insertion or deletion mutations can be constructed while simultaneously testing for substitution mutations. The set of queried mutations may be expanded by forming base call sets of insertion and deletion tiling probes. The difference analysis may be performed for insertions and deletions similarly to the analysis of substitutions. See Figure 9, which shows the detection of an insertion in the p53 gene. Mutation scans may be performed simultaneously for substitution, deletion and insertion probe sets. These scans can readily identify a substitution in one target and an insertion in another. See Figure 9.

### (iii) Characterizing Homozygous and Heterozygous Mutations

Many mutations can exist in either homozygous or heterozygous forms. The methods of the present invention can detect both homozygous and heterozygous mutations. Ordinarily, polynucleotide from the mutated chromosome always encounters probes in a complete or substantially complete array with which it can hybridize, and thus always will produce an overall pattern distinct from that of the reference polynucleotide. However, in a heterozygous mutation, polynucleotide from the non-mutated chromosome of the target can match the reference polynucleotide. These distinctions can be detected using "perfect match" (PM) plots. In a PM plot, the degree of hybridization of both the target and reference polynucleotides in those base call sets that are perfect matches for the reference polynucleotide are compared.

Figure 10 shows PM plots that compare homozygous and heterozygous scenarios in a 1079 bp region of the CFTR gene. Figure 10a shows mutation scans of a 170 bp region comparing a wild type CFTR polynucleotide sequence to a target containing a 3-bp DF508 (TTT) homozygous deletion. The perfect match (PM) difference plot displays the normalized intensity difference between the perfect match for references from the two arrays. Mutation scans are also shown for substitution (positive envelope only), insertion, and deletion probes. Interestingly, a 3-bp deletion (identified as "TTT" by the analysis software) was readily detected by the insertion scan.

In contrast to a homozygous mutation, the difference is not apparent in a PM plot involving heterozygous mutations. This is because, as discussed above, in a heterozygous mutation, there is one chromosome which still is the same as the reference chromosome and which provides polynucleotide fragments which will hybridize with the PM base call sets; thus, there is no apparent difference between the reference and the target. This is illustrated in Figure 10b. No footprint was detectable in the PM difference scan since DNA with the wild-type reference sequence is present in both samples. However, interestingly, the deletion scan correctly identified a 3-bp "TTT" deletion in the heterozygous sample.

Similarly, Figure 11 shows homozygous and heterozygous mutation scans of 2.5 kbp mitochondrial amplicons. Figure 1 la shows a 300 bp mutation scan of the homozygous sample. The scan revealed 3 single base substitutions. A total of 176 sequence differences among 8 different samples were examined. The mutation scan of the homozygous polynucleotides correctly identified over 90% of these sequence differences. Moreover, the false positive rate was quite low (< 1 per 3900 bp screened). Figure 11b shows the same 300 bp scan as Figure 11a but the sample is heterozygous. The scan revealed 3 single base substitutions, but with a decreased signal to noise ratio. In general, heterozygous mutations in the 2.5 kbp amplicons were detected with decreased sensitivity (70%-80%).

In summary, the present methods will allow the determination of mutations, for example, as homozygous or heterozygous, as well as identification of the location and type of mutation.

### e) Determination of Whether Two or More Polynucleotides of Unknown Sequence are Identical

The above-described methods can be easily adapted to determine whether two or more polynucleotides of unknown sequences are identical. For example, two polynucleotides of unknown sequences can be separately hybridized to two different identical or substantially identical arrays of complete or substantially complete n-mer probe polynucleotides. Their hybridization patterns can be compared by using the above-described methods. If the two sequences are identical, one would expect a perfect match in a PM plot. As discussed above, this methodology can be used to determine rapidly and more accurately whether polynucleotides apparently from a variety of sources are in fact from a single source, without having to know the sequence of any of the polynucleotides.

### f) Sequence-Specific End-Sampling

A method of distinguishing individual polynucleotides in a mixture of polynucleotides is also provided. Such mixture of polynucleotides may result from, for example, fragmentation of DNA targets with a site-specific DNA nuclease. Such fragmentation may produce a population of fragments representing only a small subset of all possible 5-' or 3'-terminal subsequences. N-mer arrays can be used to identify the desired terrnini in such a complex sample using the methodology already described above. The hybridization pattern observed by hybridizing a complex sample of either polynucleotides or their fragments to a complete or substantially complete n-mer array easily lends itself to determination of the number and type of the polynucleotides, or their fragments, of interest. This method of distinguishing certain polynucleotides in a complex population is much faster and more accurate compared to traditional methods such as gel-based or charge-based methods. Potential applications include the analysis of DNA fragments generated by random or directed genomic sampling strategies, such as in DNA fingerprinting or differential display.

### EXAMPLES

The following examples describe in detail the synthesis of the array, fragmentation of target and reference polynucleotides, hybridization and ligation, washing and staining of arrays, and data analysis. While these examples further clarify the methods described herein, they are not to be construed as limiting the scope of the invention.

### General Techniques

Complete n-mer deoxypolynucleotides were synthesized on derivatized glass substrates using light-directed photochemistry as previously described by Pease et al., (1994) *Proc Natl Acad Sci U S A 91,* 5022-6, herein incorporated in its entirety by reference. 8-mer arrays are synthesized with 50x50 µm probe feature sizes and 9-mers with 25x25 µm probe feature sizes. Prior to photochemical synthesis, a DMT-hexaethyleneglycol-(2-cyanoethyl-N,N-diisopropyl) phosphoramidite linker (for example, that from ChemGenes, 25 mM in acetonitrile) and 19 bases of a constant anchor polynucleotide (³'ATACGTAGACACTGCTGGAC ^{5'}) were synthesized on the array using conventional DMT PAC phosphoramidites (25 mM in acetonitrile): T b-cyanoethyl, IBU dC b-cyanoethyl, PAC dA b-cyanoethyl, and iPr-PAC dG b-cyanoethyl phosphoramidites (for example, those available from Pharmacia Biotech).

The last base of the anchor polynucleotide and the subsequent n (n = 8 or 9 in this embodiment) combinatorial bases were synthesized using light-directed photochemistry (Pease et al., 1994). Following synthesis of these n bases, two bases of inosine (2'-deoxylnosine, for example, from Glen Research) were added to the 5'terminus using conventional DMT phosphoramidite chemistry. The addition of two inosines improves overall array signal intensities without greatly affecting discrimination. After synthesis, the DNA molecules on the arrays were deprotected in a 50% ethylenediamine (EDA)/50% ethanol bath for ~ 10 hrs.

A 20-mer anchor polynucleotide (5'-psoralen-TATGCATCTGTGACGACCTG-3') complementary to the constant anchor sequence of the probe was synthesized using a standard polynucleotide synthesizer. Two versions of the polynucleotide were synthesized for the different modalities of ligation: a 3' hydroxyl version for enzymatic ligation and a 3' phosphoryl version for chemical ligation. The anchor polynucleotide also contained a AT dinucleotide pair and a psoralen C6 (for example, from Glen Research) moiety at the 5' end for use in an optional cross-linking protocol. The polynucleotide was cartridge purified via the hydrophobic 5' psoralen moiety. Anchor polynucleotides with terminal G 3'-bases were used. The actual array-based duplex probes were created by hybridizing the complementary 20-mer anchor polynucleotide (500 nM) to the constant portion of the array-bound probes.

The hybridization conditions are as follows: 6X SSPE buffer (pH 7.4) at 40° C for 1-2 hr followed by washing on a GeneChip fluidics station (of the type for example, from Affymetrix, Inc.) with 6X SSPE at 22 °C. Under the ligation conditions employed in this embodiment (30° C -40° C), psoralen cross-linking was unnecessary for maintenance of duplex stability.

eDouble stranded DNA targets (~50-100 fmols, ~ 80-160 ng of a 2.5 kb target) were randomly fragmented with DNaseI (0.5U/20 µl) in buffer (10 mM Tris-acetate, pH 7.5, 10 mM magnesium acetate, 50 mM potassium acetate) for 15 min. at 37° C. DNaseI was heat inactivated and the sample denatured by incubation at 95° C for 15 min. The sample was cooled on ice, and 3' end-labeled with terminal dideoxynucleotidyl transferase (5 U/20 µl) (TdT) (GibcoBRL, Gaithersburg MD) and ddATP-N6-biotin (25 µM) (NEN Life Sciences, Boston MA) at 37° C for 60 min. TdT was heat inactivated and the DNA denatured by incubating at 95° C for 10 min. The denatured DNA was cooled on ice, added to the ligation mix (total volume of 200 µl, final DNA target concentration of ~500 pM, see below), spun at 14000g in a microfuge for 3 min. to pellet any precipitate present, and applied to the DNA array. The ligation solution was continuously mixed across the surface of the array by rotation (40-50 r.p.m.) on a rotisserie located in an incubator set to the desired temperature.

Either enzymatic or chemical ligation was used. Each produced satisfactory results when used according to the specified protocols. The enzymatic ligation conditions are given for the following enzymes: T4 DNA ligase, *E. coli* (NAD-dependent) DNA ligase, and Taq DNA ligase. The standard T4 DNA ligation buffer consisted of: 50 mM Tris-HCl (pH 7.8), 10 mM MgCl₂, 10 mM DTT, 1 mM ATP, 50 µg/ml RSA, 100 mM NaCl, 0.1% TX-100 and 2.0 U/µl T4 DNA ligase (New England Biolabs, Inc.). *E, coli* DNA ligase buffer consisted of: 40 mM Tris-HCl (pH 8.0), 10 mM MgCl₂, 5 mM DTT, 0.5 mM NADH,0.5 µg/ml BSA, 0-1 % TX-100, and 0.025 U/µl *E*. *coli* DNA ligase (Amersham).

Taq DNA ligation buffer consists of: 20 mM Tris-HCl (pH 7.6), 25 mM potassium acetate, 10 mM magnesium acetate, 10 mM DTT, 1 mM NADH, 50 µg/ml BSA, 0.1% Triton X-100, 10% PEG, 100 mM NaCl, and 1.0 U/µl Taq DNA ligase (New England Biolabs). For both 8-mer and 9-mers, T4 and *E. coli* DNA ligase reactions were performed at 30°C, and Taq DNA ligase reactions were performed at 37-40 °C (due to the low activity of Taq at 30 °C). The ligation reactions were typically incubated overnight (14-16 hours), but incubations as short as 2 hours also gave satisfactory results.

During chemical ligation, 3-4 M tetramethylammonium chloride (TMACl) was used in the ligation buffer in order to help normalize the stabilities of A/T-rich and G/C-rich hybrids. See, Wood et al. Proc. Natl. Acad. Sci. U.S.A. 82, 1585-1588 (1985), herein incorporated in its entirety by reference. Freshly dissolved 1-ethyl-3-(3-dimethylaminopropyl)carbodiimide hydrochloride (EDC,Pierce Biochemicals) (2 M in H₂O for 10X stock) was used to generate a pyrophosphate bond between a 5' phosphoryl moiety of the target and a 3' phosphoryl moiety on the complementary anchor oligonucleotide (added during synthesis). See, for example, Hermanson, Bioconjugate Techniques: Academic Press (1996); Shabarova, Biochimie 70: 1323-1334 (1988); Kuznetsova et al. Mol. Biol. (Mosk.) 28:290-299 (1994), all of which are herein incorporated in their entirety by reference. The chemical ligation conditions were: 50 mM 2-[N-Morpholino]ethanesulfonic acid (MES) (pH 6.0 with KOH), 10 mM MgCl₂, 0.001% SDS, 200 mM EDC, 50 mM imidazole (pH 6.0 with FICI) and 3.0-4.0 M tetramethylammonium chloride (TMACl) (Sigma) for 14 hrs at 30 °C.

Following ligation, arrays were washed 5-10 times with 1X SSPE (pH 7.4, 22°C) on a GeneChip^{TM} fluidics station, stained for 5 min. with streptavidin-phycoerythin conjugate (Molecular Probes, 2 ng/µl in 1X SSPE, 50 µg/ml BSA) on a rotating rotisserie at 22° C and washed another 5-10 times with 1X SSPE.

The fluorescent signal from the array was detected using specially designed confocal scanners (Affymetrix, Inc., Santa Clara, CA). See Chee, et al. *Science* 274, 610-614 (1996), herein incorporated by reference. The scanners image the 8-mer arrays at 6.8-7.5 µm resolution, and image 9-mer arrays at 3.5 µm resolution. Images were processed with GeneChip^{TM} software (Affymetrix Inc., Santa Clara, CA), and the resultant intensity data was transformed into a text file containing an alphabetized list of probe sequences and corresponding intensities. These data were then analyzed by custom software written in Visual Basic^{TM} (v. 4.0, Microsoft).

### Example 1: The Relationship Between Base Call Accuracy, and Probe and Target Lengths

As a threshold matter, the dependency of base call accuracy on either target length or probe length should be determined. The methods were used to sequence polynucleotides of various lengths, then the called sequences were compared to the actual, known sequence. DNA sequences of different lengths were hybridized and ligated to both 8-mer and 9-mer probe arrays using enzymatic and chemical ligation. A variety of sequences were analyzed, including a 535 bp PCR product from the human CFTR gene; a 1.2 kbp PCR product from HIV-1 *pol* gene; a 2.5 kbp PCR fragment spanning the mitochondrial *cytochrome* b *gene* and control region; and the 5.4 kbp genome of the bacteriophage φX174.

The results demonstrate that target length and probe length significantly affected the base calling accuracy, See Table 1. As target length increased, base calling accuracy decreased from 100% (with 9-mers) at 535 bp to 89% at 5.4 kbp. By employing a confidence threshold, the number of incorrect base calls was kept to a minimum. In the case of the 5.4 kbp target called at 89% accuracy, the remaining 11% of the base calls consisted of 10.6% no calls and only 0.4% incorrect calls (false positives). Moreover, for the 89.4% (89.0% + 0.4%) of the bases actually called, the correct call rate is 99.4% (100%^{^{‡}}89.0/89.4). Thus the classification of base calls into call/no call categories greatly increased the usefulness of this base call information.

In general, the decrease in base calling accuracy with increasing target length is primarily due to no calls resulting from a lack of probe uniqueness. Increasing the probe length dramatically reduces the number of no calls and leads to a much higher base calling accuracy. Note in particular the contrast in base-call accuracy with an 8-mer vs. 9-mer probe for a 5.4kbp target, as shown in Table 1 below. On the other hand, the mode of ligation had only a modest effect, with enzymatic ligation producing a slightly higher base calling accuracy than chemical ligation in most cases.

**Table 1.**

| **Base calling accuracy as a function of target complexity, probe length, and ligation method** | | | | | |
|---|---|---|---|---|---|
| | | 540 bp CFTR | 1.2 kbp HIV | 2:5 kbp Mito | 5.4 kbp ΦX174 |
| Enzymatic | 8-mer (%) | 99.0 (0.2) | 98.2 (0.1) | 88.9 (1.3) | 73.9 (4.1) |
| | 9-mer (%) | 99.8 (0.0) | 99.9 (0.0) | 94.5 (0.3) | 85.2 (0.5) |
| Chemical | 8-mer (%) | 99.0 (0.4) | 96.1 (0.5) | 87.8 (1.5) | 69.0 (2.8) |
| | 9-mer (%) | 100.0 (0.0) | *99.5 (0.0) | 93.4 (0.) | 89.0 (0.4) |
| Incorrect call rates are shown in parentheses. Enzymatic ligation employed standard T4 DNA ligase conditions. A confidence threshold of 1.15 was used for the base calling. No call rate + incorrect call rate + base call accuracy = 100%. ∗Sdev = 0.1% (n = 4 experiments). | | | | | |

### Example 2: Substitution Mutation Characterization in HIV-1 pol gene

The HIV-1 *pol* gene was used as an example of a gene subject to substitution mutations. For purposes of illustration, two 1.2 kbp amplicons of the gene were analyzed.

First, a single substitution mutation was analyzed. Traces were generated for an A → G base substitution in two different 1.2 kbp HIV samples (a reference and a target sample) ligated to separate 8-mer arrays, constructed as described above. See Figure 6. The samples were fragmented and ligated to separate 8-mer arrays using the techniques described above, and the 8 base call sets interrogating position 435 within the reference sequence were analyzed. The intensities of the G-query probes increased and the A-query probes decreased (target relative to reference) indicating an A → G base substitution.

In this example the first two base call sets do not indicate a difference; this is probably due to binding of other subsequences in the samples to these probes. However, because of the high degree of redundancy for each base, the other base call sets for position 435 remove the systematic base calling "error" generated by the first two base call sets. The substitution mutation is easily characterized by examining the 12-14 bases around the mutation site, because tne mutation leaves a characteristic "footprint" centered around the mutation site (A435G). This is illustrated in Figure 7c.

In a further experiment, two 1.2 kbp HIV polynucleotides (a reference and a target) were fragmented and ligated to a standard probe array. As shown in Figure 8, 11/11 mutations were correctly identified by the mutation scan with no false positives. A potential false positive mutation was observed as a narrow footprint at position 770 (denoted by an asterisk in Figure 8). However this footprint fell below the pre-selected threshold, and more importantly, the footprint did not exhibit the broad profile characteristic of an actual mutation.

A third example using the HIV gene demonstrates that the present invention is also suitable to detect sequence differences between a more diverse set of targets. A mutation scan of a second 1.2 kbp HIV variant sample containing 27 single base substitutions was performed. On an 8-mer array, 96.3% (26/27, 1 false positive) of the mutations were detected, and on a 9-mer array, 100% (27/27, 1 false positive) of the mutations were detected. It should be noted that as the interrogated target sequence diverges from the reference sequence, the probability of a false positive increases due to the greater number of probes affected by the base changes. These results indicate that kilobase sized targets differing by as many as 1 in 40 bases can be accurately characterized with this approach.

### Example 3: Substitution Mutation Characterization in Longer Polynucleotide Sequences (Mitochondrial DNA)

Eight different 2.5 kbp human mitochondrial *cytochrome b* amplicons for 176 known sequence differences were screened. Using 8-mer arrays, greater than 90% (160/176) of the sequence differences were correctly identified, with a low false positive rate of less than 1 per 3900 bases (<0.02 %). Without the comparative approach *(i.e.,* by comparing separately determined sequences), the false positive rate was considerably higher (~ 1%-2%). False negatives (undetected mutations) usually occurred in target regions generating low probe signal intensities *(e.g.,* AT-rich regions) or in regions containing repeat sequences. The maximum target length that can be effectively screened with this approach depends on a tradeoff between the number of tolerated false positives (incorrectly identified as a mutation) and false negatives (undetected mutations). Thus, for purposes of mutation discovery, an 8-mer array can be used to scan targets 2.5 kbp in length with high sensitivity (typically greater than 90%) and a minimal number of false positives (fewer than one per 3900 bases).

Screening even longer pieces of DNA was explored using the entire 16.6 kbp human mitochondrial genome. Two 16.6 kbp targets consisting of three overlapping PCR amplicons from two different individuals were used. To improve sequence resolution, the prepared targets were ligated to 9-mer arrays. The mutational screen identified over 66% (16/24) of the known sequence differences with a low false positive rate of 4 per 16,600 bases (0.024%). These results show that comparative resequencing on complete n-mer DNA arrays can be used to rapidly screen very complex DNA samples for mutations.

### Example 4: Insertion and Deletion mutation Characterization in p53 Gene.

Base calling and virtual tiling methods described herein allow one to examine a gene not only for substitution mutations, but for insertion and deletion mutations as well. To illustrate this aspect, the set of queried mutations were expanded by forming base call sets of insertion and deletion tiling probes. The difference analysis was performed for insertions and deletions similarly to the analysis of substitutions. An unknown homozygous p53 (11 exons) target (applied to one array) was compared to a wild-type p53 reference (applied to a second array). Mutation scans were performed for substitution, deletion and insertion probe sets. Figure 9 shows the mutation scans. These scans readily identified a G→C substitution in exon 4 and a C insertion in exon 5. See Figure 9a.

The mutation scan of exon 5 on the other hand showed footprints in both the substitution and insertion scans. See Figure 9b. The presence of footprints in both the substitution and insertion scans of exon 5 led to difficulty in deciding which type of mutation was present. However, previous analysis of HIV samples containing single-base substitutions indicated that authentic substitution mutations typically did not display footprints in insertion or deletion scans. Thus the observation of an insertion footprint in the exon 5 scan strongly supports the insertion call, which was confirmed by conventional sequencing.

### Example 5:Characterizing Homozygous and Heterozygous Mutations in CFTR Gene

The ability of 8-mer arrays to detect homozygous and heterozygous mutations in cystic fibrosis transmembrane conductance regulator (CFTR) amplicons (~1.1 kbp) containing a known ΔF508 deletion (3 bp) was investigated. A wild type (normal) CFTR sample was used as the reference. The mutation scans are shown in Figure 10. The ΔF508 deletion was readily detected in the homozygous sample by the presence of a footprint in the PM reference probe scan (a scan of the difference in perfect match reference probes), substitution scan and deletion scan. See Figure 10a. Figure 10b shows the mutation scan for heterozygous sample. In the case of the heterozygous deletion, no footprint was seen in the PM reference probe scan since the DNA with the reference sequence is present in both samples that are being compared. However, the deletion scan still exhibited a well resolved footprint, since target containing deleted sequence is present in the heterozygous sample but completely absent from the reference sample. Thus, mutations could be characterized as a heterozygous deletion.

The method was extended to a longer "heterozygous" sample of 2.5 kbp mitochondrial amplicons. Figure 11 shows a mutation scan of 2.5kbp mitochondrial amplicons (homozygous v. heterozygous). A 300 bp mutation scan of the homozygous sample (50:50 mixture of wild type and homozygous DNAs) revealed 3 single base substitutions. See Figure 11a. A total of 176 sequence differences among 8 different samples were examined. The mutation scan of the homozygous samples correctly identified over 90% of these sequence differences. Moreover, the false positive rate was quite low (<1 per 3900 bp screened).

The same 300 bp scan was repeated, except using a "heterozygous" sample (a 50:50 mixture of wild type and heterozygous DNAs). The mutation scans are shown in Figure 11b. This scan revealed 3 single base substitutions, but with a decreased resolution. In general, heterozygous mutations in the 2.5 kbp amplicons were detected with decreased sensitivity.

### Example 6: Site-Specific End-Sampling of Bacterophage φX174

As an example of the use of the present invention for site-specific end-sampling, the bacteriophage φX174 (~5.4 kbp) was digested with the restriction endonucleases, Pall (GG↓CC) and NlaIII (CATG↓), and the resulting fragment population was ligated to an 8-mer array. The results indicated that 32 out of 33 restriction sites were detected, indicating that n-mer arrays can be used to identify 5' termini subsequences in a complex sample.

The above description is illustrative and not restrictive. Many variations of the invention will become apparent to those of skill in the art upon review of this disclosure. Merely by way of example a variety of substrates, polymers, linking groups, synthesis initiation sites, and other materials may be used without departing from the scope of the invention. The scope of the invention should, therefore, be determined not with reference to the above description, but instead should be determined with reference to the appended claims along with their full scope of equivalents.

## Claims

1. A method of determining the presence of a mutation in a target polynucleotide, comprising the steps of:
(a) providing at least two identical or substantially identical polynucleotide probe arrays, wherein each probe comprises a double stranded region and a single-stranded n-mer overhang region such that the overhangs in each array constitute a complete or substantially complete set of n-mers;
(b) hybridizing the target polynucleotide to said overhangs of probe polynucleotides in one array to generate a target hybridization pattern;
(c) hybridizing a reference polynucleotide to said overhangs of probe polynucleotides in a second array to generate a reference hybridization pattern; and
(d) determining the presence of a mutation in the target polynucleotide by comparing the reference and target hybridization patterns.

2. The method of claim 1, wherein in step b), the hybridized target polynucleotide is ligated to the probe.

3. The method of claim 1, wherein in step c), the hybridized reference polynucleotide is ligated to the probe.

4. The method of claim 1, wherein the overhangs have free 5'-ends.

5. The method of claim 1, wherein the overhangs have free 3'-ends.

6. The method of claim 1, wherein the n-mer comprises from about 4 to about 50 nucleotides.

7. The method of claim 1, wherein the mutation is a substitution mutation.

8. The method of claim 1, wherein the mutation is a deletion mutation.

9. The method of claim 1, wherein the mutation is an insertion mutation.

10. The method of claim 1, in which said target polynucleotide is selected from the group consisting of: a cystic fibrosis transmembrane conductance regulator gene, a p53 gene, a mitochondrial DNA, or an HIV gene.

11. The method of claim 1, wherein the arrays are arranged in parallel.

12. A method of determining whether two or more target polynucleotides are identical, comprising the steps of:
(a) providing at least two identical or substantially identical polynucleotide probe arrays, wherein each probe comprises a double stranded region and a single-stranded n-mer overhang region such that the overhangs in each array constitute a complete or substantially complete set of n-mers;
(b) hybridizing first target polynucleotide to said overhangs of probe polynucleotides in one array to generate a first hybridization pattern;
(c) hybridizing second target polynucleotide to said overhangs of probe polynucleotides in a second array to generate a second hybridization pattern; and
(d) comparing the first and second hybridization patterns.

13. The method of claim 12, wherein in step b), the hybridized target polynucleotide is ligated to the probe.

14. The method of claim 12, wherein in step c), the hybridized reference polynucleotide is ligated to the probe.

15. The method of claim 12, wherein the overhangs have free 5'-ends.

16. The method of claim 12, wherein the overhangs have free 3'-ends.

17. The method of claim 12, wherein the n-mer comprises from about 4 to about 50 nucleotides.

18. The method of claim 12, wherein the arrays are arranged in parallel.

19. A method of distinguishing individual polynucleotides in a mixture of polynucleotides comprising the steps of:
(a) providing at least two identical or substantially identical polynucleotide probe arrays, wherein each probe comprises a double stranded region and a single-stranded n-mer overhang region such that the overhangs in each array constitute a complete or substantially complete set of n-mers;
(b) hybridizing the polynucleotides in the mixture to said overhangs of probe polynucleotides in the array; and
(c) determining the hybridization pattern.

20. The method of claim 19, further comprising enumerating the distinguished individual polynucleotides.

21. The method of claim 19, wherein in step b), the hybridized target polynucleotide is ligated to the probe.

22. The method of claim 19, wherein the overhangs have free 5'-ends.

23. The method of claim 19, wherein the overhangs have free 3'-ends.

24. The method of claim 19, wherein the n-mer comprises from about 4 to about 50 nucleotides.

25. The method of claim 19, wherein the arrays are arranged in parallel.
